# EUROPEAN PATENT SPECIFICATION

(11) **EP 4 185 309 B1**
(45) Date of publication and mention of the grant of the patent: **01.07.2026**
(21) Application number: 21754740.5
(22) Date of filing: 23.07.2021
(51) Int. Cl.: A61K 35/745, A61K 35/747, A61P 3/00, A61P 3/04, A61P 3/06, A61P 3/10, A61P 9/00, A61P 13/12, A61P 19/06

(54) **A COMPOSITION COMPRISING LACTOBACILLUS STRAINS FOR TREATING OR PREVENTING CONDITIONS OF ELEVATED SERUM URATE**
EINE ZUSAMMENSETZUNG AUS LACTOBACILLUS-STÄMMEN ZUR BEHANDLUNG ODER VORBEUGUNG VON ERKRANKUNGEN MIT ERHÖHTEM SERUMURATSPIEGEL
COMPOSITION COMPRENANT DES SOUCHES DE LACTOBACILLUS POUR LE TRAITEMENT OU LA PRÉVENTION DES ÉTATS D'HYPERURICÉMIE.

(30) Priority: 24.07.2020 CH 9232020
(43) Date of publication of application: 31.05.2023
(73) Proprietor: Beo Therapeutics, 8030 Zürich (CH)
(72) Inventor: NIELSEN, Rikke Christina, 8038 Zürich (CH)
(74) Representative: Rentsch Partner AG
(86) International application number: PCT/EP2021/070685
(87) International publication number: WO 2022/018263

(56) References cited:
- CN-A- 108 486 007
- CN-B- 110 055 199
- US-A1- 2013 330 299
- US-A1- 2017 106 029
- US-A1- 2017 216 376
- US-A1- 2019 309 269
- LEI KAI ET AL: "Effect of dietary supplementation ofBacillus subtilisB10 on biochemical and molecular parameters in the serum and liver of high-fat diet-induced obese mice", SCIENCE B: INTERNATIONAL BIOMEDICINE & BIOTECHNOLOGY, ZHEIJIANG UNIVERSITY PRESS, CN, vol. 16, no. 6, 25 May 2015 (2015-05-25), pages 487 - 495, XP036297868, ISSN: 1673-1581, [retrieved on 20150525], DOI: 10.1631/JZUS.B1400342
- TRIPATHI SHIKHA ET AL: "Structure-Based Immunogenicity Prediction of Uricase from Fungal (), Bacterial () and Mammalian Sources Using Immunoinformatic Approach", PROTEIN JOURNAL, KLUWER ACADEMIC/PLENUM PUBLISHERS, DORDRECHT, NL, vol. 39, no. 2, 27 March 2020 (2020-03-27), pages 133 - 144, XP037093815, ISSN: 1572-3887, [retrieved on 20200327], DOI: 10.1007/S10930-020-09886-0
- RADI R ET AL: "Inhibition of xanthine oxidase by uric acid and its influence on superoxide radical production", BIOCHIMICA ET BIOPHYSICA ACTA. PROTEIN STRUCTURE AND MOLECULAR ENZYMOLOGY, ELSEVIER, AMSTERDAM; NL, vol. 1122, no. 2, 31 July 1992 (1992-07-31), pages 178 - 182, XP023579831, ISSN: 0167-4838, [retrieved on 19920731], DOI: 10.1016/0167-4838(92)90321-4

## Description

### Field of the Disclosure

The present disclosure relates to compositions comprising a plurality of microorganisms for use in the reduction of blood urate concentration and thus for the therapeutic and the preventive treatment of subjects at risk or suffering from elevated blood urate levels and their use in form of pharmaceutical compositions or nutraceutical compositions for treatment and prevention of urate and hyperuricemia associated diseases.

### Background.

Urate is the final product of purine nucleoside metabolism. It is secreted both via the renal system and via the intestines. The final steps in the metabolism of purines into urate is the target of multiple drugs and the reactions are catalysed by the enzyme xanthine oxidase. Xanthine oxidase is primarily expressed in the liver, small intestines and by the gut microbiome. Urate is in most mammals further metabolised to allantoin by the enzyme uricase. Humans do not express the gene for uricase, and the human intestinal microbiome is responsible for metabolising urate to allantoin.

Urate is the monovalent sodium salt of uric acid. Urate has low solubility and forms crystals in serum at concentrations of 6.8 mg/dl (405 µmol/L) (Burns et al, 2012. Chapter 359. Disorders of purine and pyrimidine metabolism. Harrison's Principles of Internal Medicine, 1 8e New York, NY: McGraw-Hill). The crystals can be deposited throughout the body and give rise to inflammatory responses as seen in gout patients or kidney damage as seen in patients with chronic kidney disease.

Abnormally high concentrations of urate or uric acid in blood, also referred to as hyperuricemia, is known to be associated with a number of diseases and conditions, including cardiovascular disease, metabolic syndrome, non-alcoholic fatty liver disease, chronic kidney disease, gout, insulin resistance, hypertension, dyslipidaemia, renal insufficiency, obesity, pre-diabetes and diabetes, particularly type II diabetes.

Elevated levels of urate can be the result of an increased production of uric acid by xanthine oxidase or a decreased urate excretion via the intestines, the renal system, or a combination of both. The renal system excretes two thirds of the total urate in healthy humans. The intestines complement the renal system and excrete one-third of the total urate. The excretion of endogenous urate through the intestinal tract is an opportunity for treating hyperuricemia locally thereby avoiding the systemic adverse reactions.

A variety of drugs are known to lower urate serum levels. Xanthine oxidase inhibitors (e.g. allopurinol and febuxostat) limit the formation of urate in the body by inhibiting xanthine oxidase and uricosuric agents (e.g. probenecid) increase urate elimination by the kidneys. Unfortunately, the treatment of patients with xanthine oxidase inhibitors and uricosuric agents are associated with side effects, including rashes, nausea, stomach pain, kidney stones and reduced liver function. Further, the drugs show low efficacy and only 30-40% of patients treated with allopurinol reach the treatment target.

Other treatment strategies that have been considered include a number of vitamins, such as vitamin B2, B9, C that have been associated with reduction or prevention of elevated serum uric acid.

The use of bacteria for degradation of urate has been considered in some early studies. Nine cultures of aerobic bacteria capable of growing on an elective medium containing uric acid as the only source of carbon, nitrogen and energy were identified (Rouf MA and Lomprey RF Jr. 1968. "Degradation of uric acid by certain aerobic bacteria." J Bacteriol., Sep;96(3): 617-22). However, the urate lowering effects of the were not studied. Consequently, there is a need for new treatment strategies with fewer side-effects and improved efficacy.

The present disclosure is able to overcome the drawbacks of the prior art by addressing both the over-production and the under-excretion of urate.

The microbial treatment according to the present disclosure can reduce urate concentration in blood by treating hyperuricemia locally by optimising the metabolism of the human gut microbiome and by optimising the intestinal host - microbiome interactions. More specifically, the treatment modulates two pathophysiological pathways; (1) it reduces the over-production of uric acid by inhibiting xanthine oxidase and (2) it increases the excretion of urate by increasing the degradation of urate.

CN 110 055 199 B describes a strain of Lactobacillus plantarum UA149 and its application in the preparation of products with uric acid-lowering or anti-gout functions.

CN 108 486 007 A describes a lactobacillus casei strain ZM15 for lowering the blood uric acid. Further disclosed therein is a probiotic composition obtained by compounding four strains of ZM15, ZM18, ZM122 and ZM05.

US 2013/330299 A1 describes a composition composed of a Lactobacillus bacterium, Bifidobacterium longum, and xylooligosaccharide is provided for use in reducing uric acid levels in the blood and preventing or treating hyperuricemia or gout.

US 2017/106029 A1 describes a delayed-release composition composed of a Lactobacillus bacterium, Bifidobacterium longum, Streptococcus thermophlus, inulin and xylooligosaccharide.

Lei Kai et al (Science B: International Biomedicine & Biotechnology, Zheijiang University Press, CN, vol. 16, no. 6, 25 May 2015, pages 487-495) describes the beneficial effect of probiotic Bacillus subtilis B10 on the homeostasis of the lipid metabolism and on alleviating oxidative stress in HFD-induced obese mice.

US 2017/216376 A1 describes a method for inhibiting xanthine oxidase and for reducing uric acid levels using a composition obtained by culturing Lactobacillus rhamnosus in a medium. Also disclosed is a composition including a metabolite of Lactobacillus rhamnosus for reducing uric acid levels in a subject and a method for producing the composition.

### Summary of the Disclosure

The present disclosure relates to compositions comprising bacterial strains for use in the reduction of blood urate concentration. The invention is set out in the appended set of claims.

In one aspect, the present disclosure is directed towards a composition comprising a plurality of microorganisms for use in a method of treating or preventing the condition of elevated serum urate, wherein the composition increases the gut microbiota uricase metabolic capacity and reduces xanthine oxidase metabolic capacity.

The plurality of microorganisms is a plurality of bacterial strains. The plurality of bacterial strains comprises at least one bacterial strain with xanthine oxidase inhibitory activity and at least one bacterial strain with uricase activity. In some embodiments, the plurality of bacterial strains has a Generally Recognised As Safe (GRAS) status and/or Qualified Presumption of Safety (QPS) status. In further embodiments, the plurality of bacterial strains is isolated from blood. In some embodiments, the at least one bacterial strain with xanthine oxidase inhibitory activity expresses a metabolite that inhibits xanthine oxidase. In some embodiments, the metabolite is a flavonoid. In further embodiments, the plurality of bacterial strains has a synergistic effect in lowering serum urate levels. In further embodiments, the plurality of bacterial strains has a synergistic effect in lowering serum urate levels.

The plurality of bacterial strains is selected from the following strains: *Lactobacillus casei* strain deposited with the DSMZ under deposit number DSM 33579 on July 14^{th}, 2020, *Lactobacillus plantarum* strain deposited with the DSMZ under deposit number DSM 33580 on July 14^{th}, 2020 and *Lactobacillus plantarum* strain deposited with the DSMZ under deposit number DSM 33581 on July 14^{th}, 2020. DSMZ

In some embodiments, the composition is co-formulated and/or co-administered with one or more additives including micronutrients, amino acids, prebiotics, food, food additive, dietary supplement, or medical food.

In some embodiments, the composition is co-formulated and/or co-administered with one or more micronutrients. In some embodiments, the composition is co-formulated and/or co-administered with one or more trace elements. In some embodiments, the composition is co-formulated and/or co-administered with one or more amino acids. In some embodiments, the composition is co-formulated and/or co-administered with one or more prebiotics.

In some embodiments, the composition is co-formulated and/or co-administered in combination with a further agent, e.g. a therapeutic agent and/or therapy.

In another aspect, the present disclosure is directed towards the composition according to the disclosure in form of a pharmaceutical composition or nutraceutical composition.

The condition of elevated serum urate is selected from cardiovascular disease, metabolic syndrome, non-alcoholic fatty liver disease (such as non-alcoholic steatohepatitis (NASH)), chronic kidney disease, gout, insulin resistance, hypertension, hyperuricemia, dyslipidaemia, renal insufficiency, obesity, pre-diabetes and diabetes.

### Figures

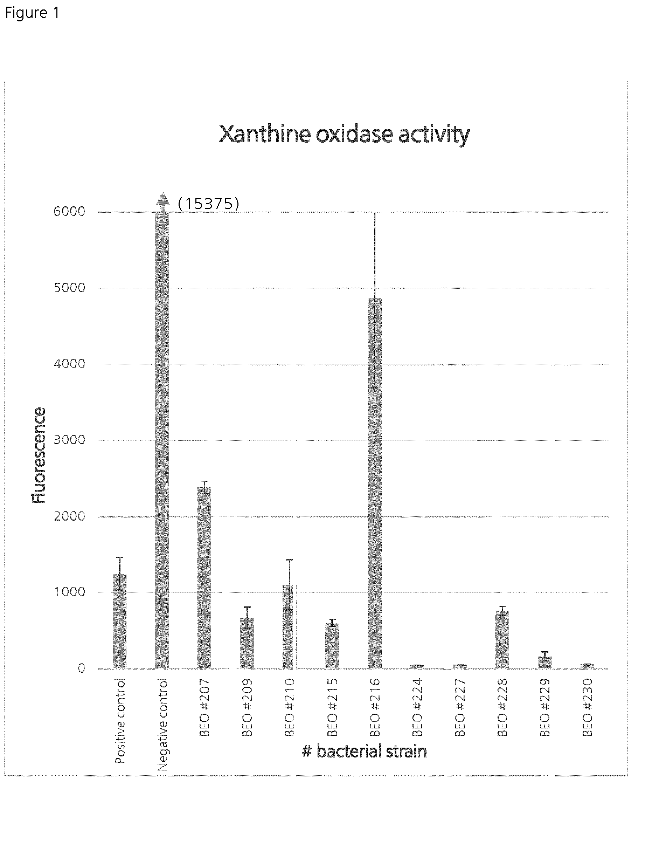

Figure 1: Figure 1 shows the fluorescence (measured in fluorescence units using excitation at 530±1 2.5 nm and fluorescence detection at 590±17.5 nm) after subjection of the corresponding bacterial strains to the enzyme assay Amplex^{®} Red Xanthine/Xanthine Oxidase Assay Kit (Catalog No. A22182, Molecular Probes), as described in detail in example 2; for specification of the bacterial strain abbreviations, see table 1; *Bifidobacterium breve* strain ATCC 15701 was used as positive control and *Bifidobacterium animalis* (BB12^{®}) was used as negative control.

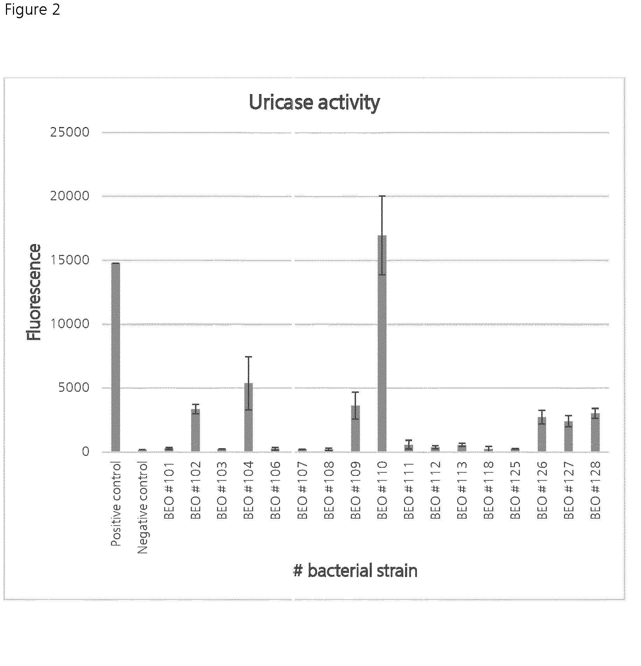

Figure 2: Figure 2 shows the fluorescence (measured in fluorescence units using excitation at 530±1 2.5 nm and fluorescence detection at 590±17.5 nm) after subjection of the corresponding bacterial strains to the enzyme assay Amplex^{®} Red Uric Acid/Uricase Assay Kit (Catalog No. A22181, Molecular Probes), as described in detail in example 3; for specification of the bacterial strain abbreviations, see table 1; 20 mM H₂O₂ solution was used as positive control and 0 mM uricase solution was used as negative control.

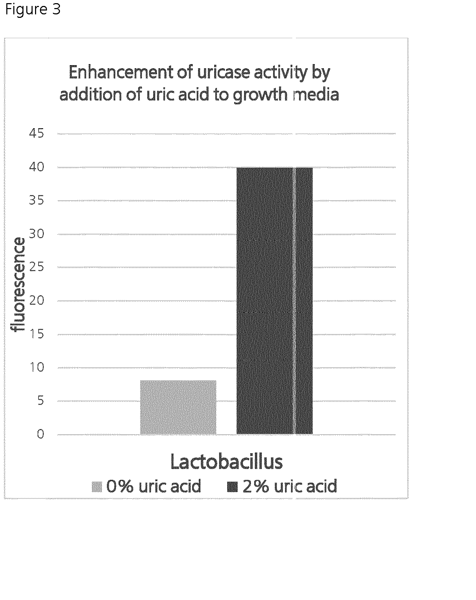

Figure 3: Figure 3 shows the fluorescence (measured in fluorescence units using excitation at 530±1 2.5 nm and fluorescence detection at 590±17.5 nm) after subjection of the bacterial strain *Lactobacillus casei* (BEO #109), which was grown on a medium in which 0% and 2% uric acid was dissolved, to the enzyme assay Amplex^{®} Red Uric Acid/Uricase Assay Kit (Catalog No. A22181, Molecular Probes), as described in detail in example 5.

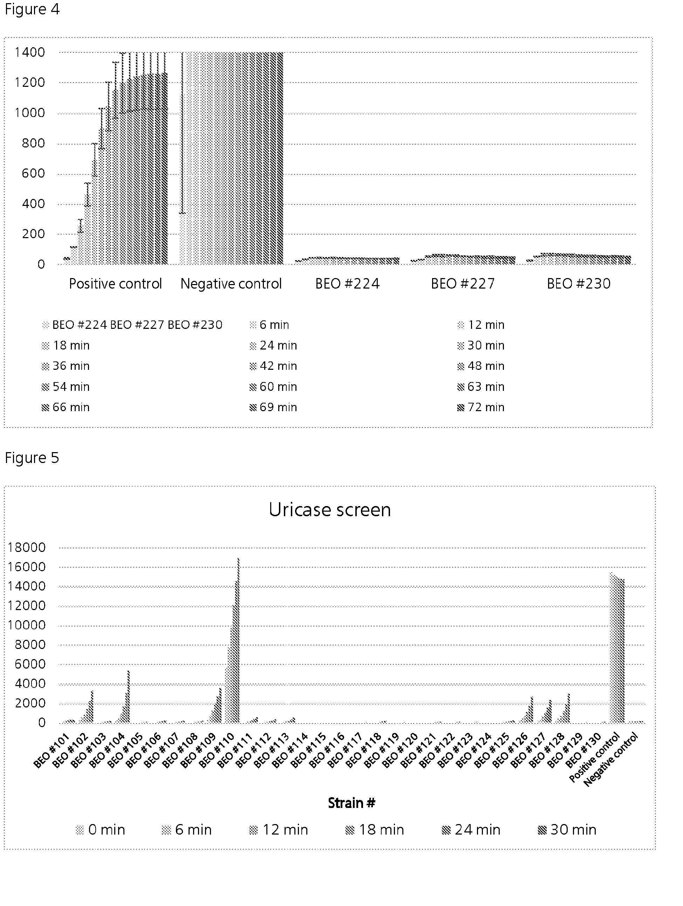

Figure 4: Figure 4 shows the fluorescence (measured in fluorescence units using excitation at 530±1 2.5 nm and fluorescence detection at 590±17.5 nm) at different points in time after subjection of the corresponding bacterial strains to the enzyme assay Amplex^{®} Red Xanthine/Xanthine Oxidase Assay Kit (Catalog No. A22182, Molecular Probes), as described in detail in example 6; for specification of the bacterial strain abbreviations, see table 1; *Bifidobacterium breve strain* ATCC 15701 was used as positive control and *Bifidobacterium animalis* (BB12^{®}) was used as negative control.
Figure 5: Figure 5 shows the fluorescence (measured in fluorescence units using excitation at 530±1 2.5 nm and fluorescence detection at 590±17.5 nm) at different points in time after subjection of the corresponding bacterial strains to the enzyme assay Amplex^{®} Red Uric Acid/Uricase Assay Kit (Catalog No. A22181, Molecular Probes), as described in detail in example 7; for specification of the bacterial strain abbreviations, see table 1; 20 mM H₂O₂ solution was used as positive control and 0 mM uricase solution was used as negative control.

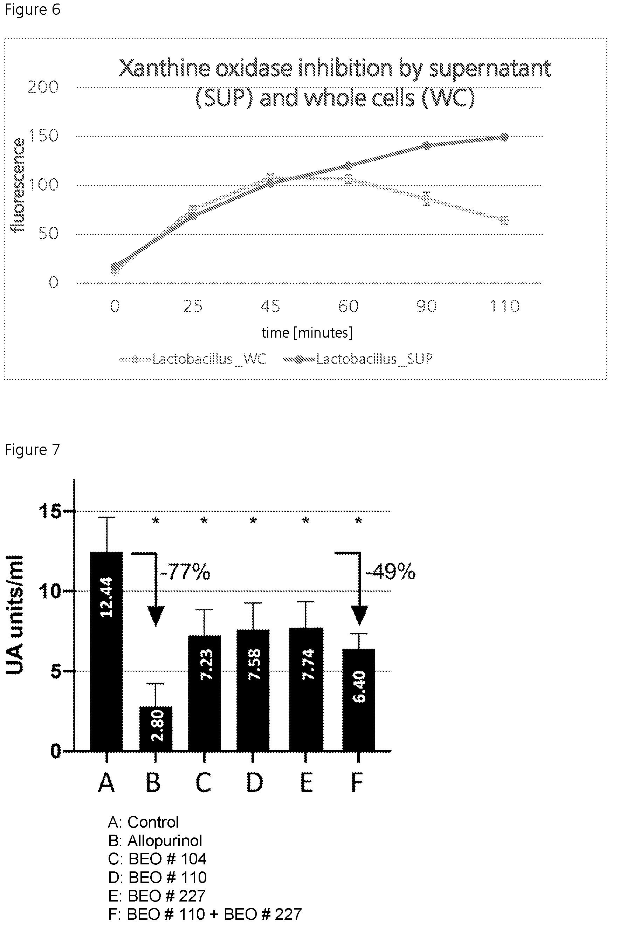

Figure 6: Figure 6 shows the fluorescence (measured in fluorescence units using excitation at 530±1 2.5 nm and fluorescence detection at 590±17.5 nm) at different points in time after subjection of the corresponding bacterial strains to the enzyme assay Amplex^{®} Red Uric Acid/Uricase Assay Kit (Catalog No. A22181, Molecular Probes), as described in detail in example 7; for specification of the bacterial strain abbreviations, see table 1; 20 mM H₂O₂ solution was used as positive control and 0 mM uricase solution was used as negative control.
Figure 7: Figure 7 shows the statistically significant lowering of mice serum uric acid in response treatment with single or combination test products. Allopurinol was used as reference product (x-axis: control (A), allopurinol (B) and test products, namely single strains # 104 (C), #110 (D), # 227 (E), and strain combination #110 and #227 (F); y-axis: uric acid (UA) units/ml with one unit corresponding to 10 µmol/L; normal physiological range lies within 40 to 65 µmol /L).

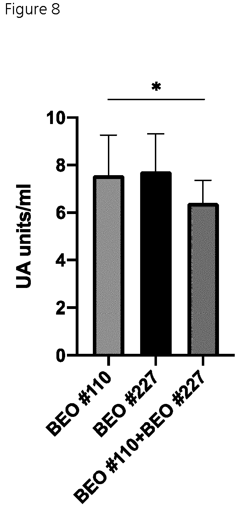

Figure 8: Figure 8 shows the statistically significant, additive effect of the administering the combination of strains #110 and #227 on the lowering of mice serum uric acid compared to administration of the single strains (x-axis: single strains #110, # 227, and strain combinations #110 and #227; y-axis: uric acid (UA) units/ml with one unit corresponding to 10 µmol/L; normal physiological range lies within 40 to 65 µmol/L).

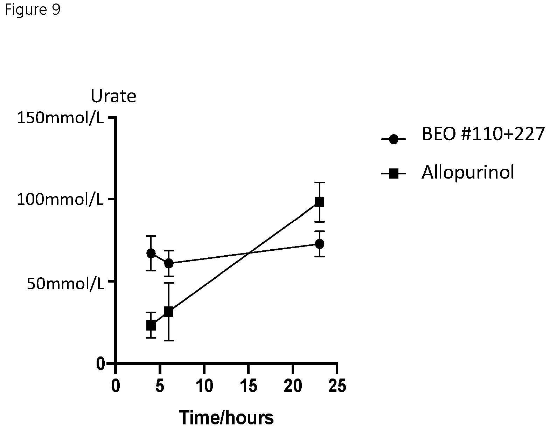

Figure 9: Figure 9 shows the effect of the bacterial treatment with the combination of strains #110 and #227 (filled black circles) compared to standard drug treatment with allopurinol (filled black squares). It was observed that the group treated with strain combination #110/#227 maintained the serum urate concentration within the normal range of 40-65 mmol/L over a 23-hour treatment cycle, whereas large fluctuations in serum urate outside the normal range was observed for the allopurinol treatment control group (x-axis: time (hours), y-axis: serum urate concentration (µmol/L).

### Detailed Description

The following definitions apply unless indicated otherwise.

The term "microorganism" as used herein refers to a single cell organism and includes a prokaryotic organism, e.g., bacteria, and a eukaryotic organism (e.g., fungi, yeast). The term bacteria includes both gram positive bacteria and gram negative bacteria, and includes but is not limited to strains from the genus *Bifidobacterium, Bacillus,* and *Lactobacillus,* e.g. strains from the species *Bifidobacterium breve, Bifidobacterium longum, Bifidobacterium adolescentis, Bifidobacterium bifidum, Bacillus subtilis, Lactobacillus plantarum, Lactobacillus rhamnosus, Lactobacillus bulgaricus, Lactobacillus fermentum,* and *Lactobacillus casei.* The selection of one or more suitable microorganism lies within the knowledge of the skilled person.

The term "strain" refers to progenies of a pure, isolated culture and the succeeding descendants that can be cultured from it without contamination. A strain refers to a sub-variety of a microbe that is phenotypically and/or genotypically distinguishable from other microbes.

The term "hyperuricemia" as used herein characterizes abnormally high concentrations of urate or uric acid in blood. Hyperuricemia is known to be associated with a number of diseases and conditions, including cardiovascular disease, metabolic syndrome, non-alcoholic fatty liver disease (such as non-alcoholic steatohepatitis (NASH)), chronic kidney disease, gout, insulin resistance, hypertension, dyslipidaemia, renal insufficiency, obesity, pre-diabetes and diabetes, particularly type II diabetes.

The term "micronutrient" includes e.g. vitamins and trace elements. Vitamins are organic substances not synthesized by the body and necessary for normal metabolism. They are divided into water soluble or fat soluble and those with or without coenzyme function. Typical vitamins for use in the present disclosure include, but are not limited to, vitamin B2, vitamin B9, vitamin C. Trace elements are metals present in very minute quantities in the body. They are essential for normal metabolic functions and are typically cofactors of enzymes or form an integral part of the structure of specific enzymes. Typical trace elements for use in the present disclosure include, but are not limited to zinc, manganese, iron, copper, molybdenum, chlorine, nickel, boron.

The term "amino acid" refers any of the twenty standard amino acids, i.e., glycine, alanine, valine, leucine, isoleucine, methionine, proline, phenylalanine, tryptophan, serine, threonine, asparagine, glutamine, tyrosine, cysteine, lysine, arginine, histidine, aspartic acid and glutamic acid, single stereoisomers thereof, and racemic mixtures thereof. The term "amino acid" can also refer to the known non-standard amino acids, e.g., 4-hydroxypro-line, ε-N,N,N-trimethyllysine, 3-methylhistidine, 5-hydroxylysine, O-phosphoserine, γ-carboxyglutamate, γ-N-acetyllysine, ω-N-methylarginine, N-acetylserine, N,N,N-tri-methylalanine, N-formylmethionine, γ-aminobutyric acid, histamine, dopamine, thyroxine, citrulline, ornithine, β-cyanoalanine, homocysteine, azaserine, and S-adenosylmethi-onine. In some embodiments, the amino acid is glutamate, glutamine, lysine, tyrosine or valine. In some embodiments, the amino acids are arginine and citrulline.

The term "prebiotics" is used for selectively fermented ingredients that result in specific changes in the composition and/or activity of the gastrointestinal microbiota, thus conferring benefit(s) upon host health. The term "prebiotics" includes but is not limited to fructooligosaccharides (FOS), inulins, galactooligosaccharides (GOS), resistant starch, pectin, beta-glucans, and xylooligosaccharides.

The term pharmaceutical compositon refers to a dosage form comprising a composition of the disclosure together with a pharmaceutically acceptable excipient.

The term "nutraceutical composition" refers to a dosage form comprising a composition of the disclosure together with a food, food additive, dietary supplement, or medical food.

The term "food" refers to any processed, semi-processed, or raw substance, which is intended for consumption by mammals, e.g animals or humans. It does not include substances intended only as pharmaceuticals. The term "food additive" as used in the present disclosure refers to an additive that is, added to, mixed with, or infiltrated into a food in the process of manufacturing the food or for the purpose of processing or storing the food, such water-binding agents, gelling agents, thickeners, antioxidants, dyes, flavor enhancers, acidulants and sweeteners (including additives to which an E (Europe) number is assigned).

The term "dietary supplement" refers to a dosage form comprising a composition of the disclosure together with a nutritional substance or a substance with a nutritional or physiological effect whose purpose is to supplement the normal diet, such as an herb or other botanical; a metabolite, an extract.

The term "medical food" refers to a dosage form comprising a composition of the disclosure together with a food intended for the dietary management of a disorder or disease to be administered under the supervision of medically trained people. Medical foods typically fulfil regulatory requirements, including, among others, those of the Federal Food, Drug, and Cosmetic Act and those established for "foods for special medical purposes" by the European Food Safety Authority. Medical foods are typically specially processed or formulated and intended to be used under medical supervision. Medical foods include, but are not limited to, oral rehydration products, nutritionally incomplete formulas, nutritionally complete formulas and formulas for metabolic disorders.

The term "co-formulation" as used herein refers to combining a composition of the disclosure with one or more additives to form a single pharmaceutical composition or single nutraceutical composition. A co-formulation is thus intended for a simultaneous administration.

The term "co-administration" as used herein refers to a combined administration of a composition of the disclosure with one or more additives. The term "combined" refers to both a simultaneous and a sequential administration (independent of the order).

The abbreviation "CFU" denotes the unit "colony-forming unit", which is commonly used in microbiology to estimate the number of viable cells of a microorganism, e.g. a bacterial strain, in a given sample. Determination of the CFU count typically involves, after an optional initial dilution, counting at least part of the number of colonies of a microorganism, e.g. a bacterial strain, on a petri dish and, based on this count, approximating the total number of viable cells in the given sample.

An enzyme metabolic capacity corresponds to the ability to provide for the conversion of at least one of the enzyme's substrates into at least one of the corresponding products and may, for example, be defined as the number of molecules of at least one substrate converted into at least one of the corresponding products in a given period of time. The enzyme metabolic capacity is influenced, among many other factors, by the amount of the enzyme present, the activity of the enzyme or isozyme present and the presence and/or absence of activators and/or inhibitors of the enzyme.

In a first aspect, the present disclosure is directed towards a composition comprising a plurality of microorganisms for use in a method of treating or preventing the condition of elevated serum urate, wherein the composition increases the gut microbiota uricase metabolic capacity and reduces xanthine oxidase metabolic capacity.

The composition increases the gut microbiota uricase metabolic capacity and reduces the gut microbiota xanthine oxidase metabolic capacity.

The plurality of microorganisms is a plurality of bacterial strains. The plurality of bacterial strains comprises at least one bacterial strain with xanthine oxidase inhibitory activity and at least one bacterial strain that displays uricase activity. Preferably, the at least one bacterial strain with xanthine oxidase inhibitory activity expresses a metabolite that inhibits xanthine oxidase. In some embodiments the metabolite is a flavonoid.

In further embodiments, the gut microbiota uricase metabolic capacity is enhanced in the presence of uric acid. In some embodiments, the enhancement is due to induction of expression of uricase in the presence of uric acid. In preferred embodiments, this enhancement amounts to a factor of 2-10, particularly 3-8.

The enhancement of the gut microbiota uricase metabolic capacity in the presence of uric acid is advantageous because it means that uricase metabolic capacity is only minimal in the absence of uric acid, thus minimizing undesired side effects in the absence of uric acid, which is associated with enhanced safety. Typically, the bacteria employed have been granted QPS (qualified presumption of safety) status by the European Food Safety Authority (EFSA) and/or GRAS (generally recognized as safe) status by the FDA.

The plurality of bacterial strains is selected from the genus *Lactobacillus.*

In some preferred embodiments, the compositions of the present disclosure comprise one or more bacterial strains selected from the species *Bifidobacterium breve, Bifidobacterium longum, Bacillus subtilis, Lactobacillus casei, Lactobacillus plantarum* and one or several other strains of intestinal bacteria or bacteria derived from food-sources, including strains of other bacterial species.

The plurality of bacterial strains from the genus *Lactobacillus* is selected from the strains DSM 33579, DSM 33580, DSM 33581 deposited with the DSMZ (International Depositary Authority: Deutsche Sammlung von Mikroorganismen und Zellkulturen GmbH, Inhoffenstrasse 7 B, 38124 Braunschweig, Germany) on July 14th, 2020.

Typically, the bacterial strains are isolated from food. They may be in liquid, frozen or dried form.

In some embodiments the metabolite is a phenolic compound and/or an oxidative derivative thereof.

In some embodiments the metabolite is a phenolic compound and/or an oxidative derivative thereof.

In some embodiments the compositions of the disclosure may be in form of a pharmaceutical composition or a nutraceutical composition.

Suitable dosage forms include but are not limited to a tablet, pill, powder, lozenge, sachet, cachet, elixir, suspension, emulsion, solution, syrup, aerosol (as a solid or in a liquid medium), ointment containing, for example, up to 10% by weight of the active component, soft capsule, hard capsule, gel-cap, tablet, suppository, solution, or packaged powder. Pharmaceutically acceptable excipients suitable for formulating the dosage form of the present disclosure include, but are not limited to, disintegrants, diluents, plasticizers, binders, glidants, lubricants, sweeteners, flavoring agents, anti-caking agents, anti-microbial agents, antifoaming agents, emulsifiers, surfactants, buffering agents and coloring agents and the like or mixtures thereof. Suitable excipients include, for example, PBS, glycerol, cocoa butter, or polyethylene glycol.

In some embodiments, the composition is co-formulated and/or co-administered with one or more additives including micronutrients, amino acids, prebiotics, food, food additive, dietary supplement, or medical food, In some embodiments, the composition of the disclosure, specifically the pharmaceutical composition or nutraceutical composition of the disclosure may be co-formulated and/or co-administered with one or more micronutrients, specifically with one or more vitamin and/or one or more trace element. In some embodiments, the composition of the disclosure, specifically the pharmaceutical composition or nutraceutical composition of the disclosure may be co-formulated and/or co-administered with one or more vitamins, preferably selected from vitamin B2, vitamin B9 and vitamin C. In some embodiments, the composition of the disclosure, specifically the pharmaceutical composition or nutraceutical composition of the invention may be co-formulated and/or co-administered with one or more trace elements, such as zinc, manganese, iron, copper, molybdenum, chlorine, nickel and boron.

In some embodiments, the composition of the disclosure, specifically the pharmaceutical composition or nutraceutical composition of the disclosure may be co-formulated and/or co-administered with one or more amino acids. In some embodiments, the composition of the disclosure, specifically the pharmaceutical composition or nutraceutical composition of the disclosure may be co-formulated and/or co-administered with one or more amino acids, such as arginine and/or citrulline.

In some embodiments, the composition of the disclosure, specifically the pharmaceutical composition or nutraceutical composition of the disclosure may be co-formulated and/or co-administered with one or more prebiotics, preferably selected from fructooligosaccharides (FOS), inulins, galactooligosaccharides (GOS), resistant starch, pectin, beta-glucans, and xylooligosaccharides.

In some embodiments, the compositions of the present disclosure may be co-formulated and/or co-administered (in combination or separately, sequentially or at the same time) with a further agent, e.g. a therapeutic agent. The further agent, e.g. therapeutic agent, denotes an agent used in addition to the plurality of microorganisms. Suitable agents that may be used as further therapeutic agents include, but are not limited to
- compounds known to treat gout such as non-steroid anti-inflammatory drugs (NSAIDs), colchicine, oral corticosteroids and/or
- urate reducing drugs as allopurinol, febuxostat, probenecid, pegloticase, benzbromarone, sulfinpyrazone, and/or
- compounds used in the treatment of hypertension and chronic kidney disease such as compounds selected from the group comprising thiazide diuretics, beta blockers, angiotensin II receptor blockers, calcium channel blockers, renin inhibitors or angiotensin converting enzyme (ACE)-inhibitors, and/or
- compounds used in the treatment dyslipidemia such as statins, fibrates, niacin, bile acid sequestrants and cholesterol absorption inhibitors.

In other embodiments, the compositions of the present disclosure may be administered in combination with a further therapy.

The compositions of the present disclosure may be used for subjects having a urate blood concentration within the normal range as well as for subjects having a urate blood concentration above the normal range to treat or to prevent an elevation of the urate concentration in blood for the subject in question.

In specific embodiments, the compositions of the present disclosure may be used for treating, preventing or reducing the risk of an elevation of the urate concentration in blood of a patient and/or subject diagnosed with or suffering from cardiovascular disease, metabolic syndrome, non-alcoholic fatty liver disease (such as non-alcoholic steatohepatitis (NASH)), chronic kidney disease, gout, insulin resistance, hypertension, hyperuricemia, dyslipidaemia, renal insufficiency, obesity, pre-diabetes and diabetes.

In further embodiments, the compositions of the present disclosure, co-formulated and/or co-administered (in combination or separately, sequentially or at the same time) with a further agent, e.g. a therapeutic agent, may be used for treating, preventing or reducing the risk of an elevation of the urate concentration in blood of a patient and/or subject diagnosed with or suffering from cardiovascular disease, metabolic syndrome, non-alcoholic fatty liver disease (such as non-alcoholic steatohepatitis (NASH)), chronic kidney disease, gout, insulin resistance, hypertension, hyperuricemia, dyslipidaemia, renal insufficiency, obesity, pre-diabetes and diabetes.

The composition for use in the reduction of urate concentration in blood of the present disclosure may be administered in a concentration of 1 x 10⁶ CFU/day or more. Preferred concentrations are at least 1 x 10⁹ CFU/day, at least 1 x 10¹⁰ CFU/day, at least 1 x 10¹¹ CFU/day, at least 1 x 10¹² CFU/day, at least 1 x 10¹³ CFU/day, at least 1 x 10¹⁴ CFU/day, at least 1 x 10¹⁵ CFU/day.

### Examples

### Materials and Methods

The urate lowering effect of the present invention as claimed may be evaluated by measuring urate concentration in a blood sample, a urine sample or a saliva sample. The analysis may be in the form of a home analysis with a test stick (BerkeleyFit saliva test).

MRS refers to the De Man, Rogosa and Sharpe agar medium. It was used as commercially available from Sigma-Aldrich and was used according to the instructions of the manufacturer. BHI refers to the Brain Heart Infusion Broth. It was used as commercially available from Sigma-Aldrich and was used according to the instructions of the manufacturer. Anaerocult refers to the reagent anaerocult, which was used as commercially available from Millipore and according to the instructions of the manufacturer. Saline referes to a sodium chloride solution in water and was used as obtained from Sigma-Aldrich.

**Table 1 shows the abbreviations used for the respective bacterial strains.**

| Abbreviation | Bacterial species |
|---|---|
| Beo #101 | Lactobacillus casei |
| Beo #102 | Bacillus subtilis |
| Beo #103 | Lactobacillus casei |
| Beo #104 | Bacillus subtilis |
| Beo #105 | Lactobacillus plantarum |
| Beo #106 | Lactobacillus plantarum |
| Beo #107 | Lactobacillus casei |
| Beo #108 | Lactobacillus casei |
| Beo #109 | Lactobacillus casei |
| Beo #110 | Lactobacillus casei DSM 33579 |
| Beo #111 | Lactobacillus casei |
| Beo #112 | Lactobacillus casei |
| Beo #113 | Lactobacillus casei |
| Beo #115 | Lactobacillus plantarum |
| Beo #116 | Lactobacillus plantarum |
| Beo #117 | Lactobacillus casei |
| Beo #118 | Lactobacillus plantarum |
| Beo #119 | Lactobacillus plantarum |
| Beo #120 | Lactobacillus plantarum |
| Beo #121 | Lactobacillus plantarum |
| Beo #122 | Lactobacillus plantarum |
| Beo #123 | Lactobacillus plantarum |
| Beo #124 | Lactobacillus plantarum |
| Beo #125 | Lactobacillus plantarum |
| Beo #126 | Bacillus subtilis |
| Beo #127 | Bacillus subtilis |
| Beo #128 | Bacillus subtilis |
| Beo #129 | Lactobacillus plantarum |
| Beo #130 | Lactobacillus plantarum |
| Beo #207 | Bifidobacterium longum |
| Beo #209 | Bifidobacterium longum |
| Beo #210 | Bifidobacterium longum |
| Beo #215 | Bifidobacterium longum |
| Beo #216 | Bifidobacterium longum |
| Beo #224 | Lactobacillus plantarum DSM 33580 |
| Beo #227 | Lactobacillus plantarum DSM 33581 |
| Beo #228 | Lactobacillus plantarum |
| Beo #229 | Lactobacillus plantarum |
| Beo #230 | Lactobacillus plantarum |

### Example 1. Culturing of bacteria for screening

Strains were precultured in 1ml in a deep-well plate under the following conditions (see Table 2).

**Table 2**

| *Species* | *Medium* | *Temp (*°*C)* | *condition* | *Time (h)* |
|---|---|---|---|---|
| Lactobacillus sp. | MRS | 37 | non shaken | 20 |
| Bifidobacterium | MRS + 0,05% cystein | 37 | anaerobic jar with anaerocult | 72 |
| Bacillus sp. | BHI | 37 | non shaken | 20 |

5% Cystein solution was freshly prepared, filter sterilized and afterwards added to the medium prior to inoculation.

Glycerol stocks were prepared in a microtiter plate by mixing 120 µl grown culture and 40 µl 60% sterile glycerol solution. The resulting glycerol stocks were stored at -80°C.

### Example 2. Xanthine Oxidase inhibition

Bacterial strains were evaluated for their ability to inhibit xanthine oxidase catalytic activity. Prior to the assay, 1/100th volume from the glycerol stocks (example 1) was added to the microtiter plate containing the appropriate medium (see overview). Culturing conditions for the different species (time, temperature, medium, condition) were chosen as set out in Table 2. Growth of the bacterial strains was assessed by visual examination before harvesting for fluorescence measurements. After cultivation, 100µl of the respective medium containing the respective species was transferred into a new microtiter plate to use in the enzymatic activity assays.

The enzyme assay, Amplex^{®} Red Xanthine/Xanthine Oxidase Assay Kit; Catalog no. A22182; Molecular Probes, were used according to the instructions of the manufacturer. Fluorescence readouts were determined after 60 min using excitation at 530±12.5 nm and fluorescence detection at 590±17.5 nm. *Bifidobacterium breve* strain ATCC 15701 was used as positive control and *Bifidobacterium animalis* (BB12^{®}) was used as negative control.

Figure 1 shows the observed fluorescence, indicating inhibition of xanthine oxidase activity by the respective bacterial strains.

### Example 3. Bacterial uricase activity

Bacterial strains were evaluated for uricase catalytic activity. Prior to the assay, 1/100th volume from the glycerol stocks (example 1) was added to the microtiter plate containing appropriate medium (see overview). Culturing conditions (time, temperature, medium, aerobic or anaerobic) depend on species and can be found in Table 1. Uric acid was dissolved in medium to 2% and the medium was filter sterilized (therefore uric acid was immediately present in the medium upon culturing). Growth of the bacterial strains was assessed by visual examination (the level of viscosity of the medium) before harvesting for fluorescence measurement. After cultivation, 100µl of the respective medium containing the respective species was transferred into a new microtiterplate to use in the enzymatic activity assays.

The enzyme assay, Amplex^{®} Red Uric Acid/Uricase Assay Kit; Catalog no. A22181; Molecular Probes, were used according to the instructions of the manufacturer. Fluorescence readouts were determined after 30 min using excitation at 530±12.5 nm and fluorescence detection at 590±1 7.5 nm. 20 mM H₂O₂ solution was used as positive control and 0 mM uricase solution was used as negative control.

Figure 2 shows the observed fluorescence, indicating enhancement of uricase activity by the respective bacterial strains.

### Example 4. Bacterial species activities

The activity of the strains listed in Table 1 was determined, as described in examples 2 and 3, as percentage of maximal fluoresence from positive controls (*Bifidobacterium breve* strain ATCC 15701 was used as positive control for the screems according to example 2 and 20 mM H₂O₂ solution was used as positive control for the screems according to example 3). The strains were subsequently categorized according to their species and the mean average activity of all strains belonging to each species was calculated. Tables 3 and 4 show the average xanthine oxidase inhibitory activity and the average uricase inhibitory activity of the respective species, respectively, wherein the average inhibitory activies were categorized as low (5-30% of positive control), medium (25-75% of positive control) and high (50-100% of positive control).

**Table 3. Xanthine oxidase inhibition:**

| Species | Inhibitory activity |
|---|---|
| *Bifidobacterium breve* ATCC 1 5701 | low |
| *Bifidobacterium longum* | medium |
| *Lactobacillus plantarum* | high |

**Table 4. Uricase activity**

| Species | Inhibitory activity |
|---|---|
| *Lactobacillus casei* | high |
| *Bacillus subtilis* | medium |
| *Lactobacillus plantarum* | low |

### Example 5. Induction of uricase activity by uric acid

The bacterial strain *Lactobacillus* casei (BEO #109) was cultured according to the conditions described in example 1.0% to 2% uric acid was dissolved in medium and filter sterilized (therefore immediately present in the medium upon culturing). Growth of the bacterial strains were assessed by visual examination before harvesting for fluorescence measurement. After cultivation, 100µl is transferred into a new microtiter plate for enzymatic activity measurements.

The enzyme assay (Amplex^{®} Red Uric Acid/Uricase Assay Kit; Catalog no. A22181; Molecular Probes), was used according to the instructions of the manufacturer. Fluorescence readouts were determined after 60 min using excitation at 530±12.5 nm and fluorescence detection at 590±1 7.5 nm.

Figure 3 shows the observed fluorescence, indicating induction of uricase activity by addition of uric acid to the growth media.

### Example 6. Xanthine Oxidase inhibition is associated with microbial growth

This experiment was conducted as described in example 2. The fluorescence readouts were measured every 6 minutes from time 0 to 72 minutes.

The enzyme assay, Amplex^{®} Red Xanthine/Xanthine Oxidase Assay Kit; Catalog no. A22182; Molecular Probes, were used according to the instructions of the manufacturer. Fluorescence readouts were determined using excitation at 530±12.5 nm and fluorescence detection at 590±17.5 nm. *Bifidobacterium breve* strain ATCC 15701 was used as positive control and *Bifidobacterium animalis* (BB12^{®}) was used as negative control.

Figure 4 shows the observed fluorescence at the respective points in time.

### Example 7. Uricase activity is associated with microbial growth

This experiment was conducted as described in example 3. The fluorescence readouts were measured every 6 minutes from time 0 to 30 minutes.

The enzyme assay, Amplex^{®} Red Uric Acid/Uricase Assay Kit; Catalog no. A22181; Molecular Probes, were used according to the instructions of the manufacturer. Fluorescence readouts were determined using excitation at 530±12.5 nm and fluorescence detection at 590±17.5 nm. 20 mM H₂O₂ solution was used as positive control and 0 mM uricase solution was used as negative control.

Figure 5 shows the observed fluorescence at the respective points in time.

### Example 8. Xanthine oxidase inhibition by supernatant

Bacterial strain *Lactobacillus plantarum* (BEO #227) was used in this experiment. The experiment was conducted as described in example 2 except that, after cultivation of the respective bacterial strain, the supernatant is isolated by centrifugation (3000 rpm, 10 min, temperature 4 °C). The same volume of whole cells (WC) and supernatant (SUP) was then used in the same assay as the one used in example 2. The fluorescence was measured after 0, 25, 45, 60, 90 and 110 minutes.

The enzyme assay, Amplex^{®} Red Xanthine/Xanthine Oxidase Assay Kit; Catalog no. A22182; Molecular Probes, were used according to the instructions of the manufacturer. Fluorescence readouts were determined using excitation at 530±12.5 nm and fluorescence detection at 590±17.5 nm.

Figure 6 shows the observed fluorescence at the respective points in time for the whole cells (WC) and supernatant (SUP).

### Example 9. Hyperuricemia mouse model

6-10 weeks old mice are randomly divided into groups of 10 mice as follows, using 1.0 x 10⁸-10¹⁰ CFU for the test product:
1) Control group
2) Allopurinol treatment group
3) Microbial treatment group 1 : Test product is bacterial strain BEO #104
4) Microbial treatment group 2: Test product is bacterial strain BEO #110
5) Microbial treatment group 4: Test product is bacterial strain BEO #227
6) Microbial treatment group 6: Test product is mix of bacterial strains BEO #110 + BEO #227

The control group is fed a normal diet (laboratory chow and water ad libitum) and treated with saline (0.5 ml, sodium chloride solution in water) by intragastric administration once a day. The other groups are fed a high-purine diet (laboratory high-purine chow and water ad libitum) and administered an intraperitoneal injection of potassium oxonate 300 mg/kg once a day. The microbial treatment groups are treated daily with approximately 1.0 × 10⁹ CFU/day by intragastric administration. The strains tested all have QPS status and they are produced and formulated under laboratory conditions.

Mice are weighed day -1 for determination of mean weight of animals in the experiment and for composition of groups. Groups are mixed within cages to avoid cage effects (except for group 1 that is in a separate cage). Disease is induced day 0 by an intraperitoneal injection of oxonate (300 mg/kg).

Serum is collected once weekly and at the end of experiment. On day 0, serum was collected 6 h after dosing of the test product; on day 7, serum was collected 4h after dosing of the test product; on day 14, serum was collected 4h after dosing of the test product and 23 hours after dosing of the test product when the mice were sacrificed. The collected blood is centrifuged, and plasma separated and frozen until further analysis. Serum is analysed for uric acid concentration. The strain combination BEO #110+BEO #227 shows a beneficial lowering effect, i.e. full normalisation of the serum urate concentration.

### Example 10. Formulation of bacterial compositions

Cultured bacteria are separated from spent media by centrifugation at 3000 rpm for 10 min at 4 °C. Harvested bacteria is re-dissolved in formulation/cryo buffer. The formulation/cryo buffer is composed of 25-50mM buffer, 10-30% sugar. Alterative formulations are tested by including additives in the formulation buffer such as 1-6% ascorbate, arginine and 0.3-1 .0 mg folate.

### Example 11.Xanthine oxidase inhibition by yeast and fungi

Fungi and yeast are evaluated for their ability to inhibit xanthine oxidase catalytic activity. Prior to the assay fungi and yeast are cultured according to conditions recommended by DSMZ. Growth of the bacterial strains are assessed by visual examination. After cultivation, 100µl is transferred into a new microtiter plate for enzymatic activity assaying.

The enzyme assay (Amplex^{®} Red Xanthine/Xanthine Oxidase Assay Kit; Catalog no. A22182; Molecular Probes), was used according to the instructions of the manufac-turer. Fluorescence readouts were determined after 60 min using excitation at 530±12.5 nm and fluorescence detection at 590±17.5 nm.

### Example 12. Uricase activity by yeast and fungi

Fungi and yeast are evaluated for uricase catalytic activity. Prior to the assay fungi and yeast are cultured according to conditions recommended by DSMZ. Growth of the bacterial strains is assessed by visual examination. Uric acid is dissolved in medium and filter sterilized (therefore immediately present in the medium upon culturing). Growth of the bacterial strains is assessed by visual examination before harvesting for fluorescence measurement. After cultivation, 100µl is transferred into a new microtiter plate for enzymatic activity assaying.

The enzyme assay (Amplex^{®} Red Uric Acid/Uricase Assay Kit; Catalog no. A22181; Molecular Probes), was used according to the instructions of the manufacturer. Fluorescence readouts were determined after 60 min using excitation at 530±12.5 nm and fluorescence detection at 590±17.5 nm.

## Claims

1. A composition comprising a plurality of bacterial strains for use in a method of treating or preventing the condition of elevated serum urate, wherein the plurality of bacterial strains is selected from the following strains: *Lactobacillus casei* strain deposited with the DSMZ under deposit number DSM 33579 on July 14th, 2020, *Lactobacillus plantarum* strain deposited with the DSMZ under deposit number DSM 33580 on July 14th, 2020 and *Lactobacillus plantarum* strain deposited with the DSMZ under deposit number DSM 33581 on July 14th, 2020, wherein the plurality of bacterial strains comprises at least one bacterial strain with xanthine oxidase inhibitory activity and at least one bacterial strain with uricase activity, wherein the composition increases the gut microbiota uricase metabolic capacity and reduces xanthine oxidase metabolic capacity, wherein the condition of elevated serum urate is selected from cardiovascular disease, metabolic syndrome, non-alcoholic fatty liver disease, chronic kidney disease, gout, insulin resistance, hypertension, hyperuricemia, dyslipidaemia, renal insufficiency, obesity, pre-diabetes and diabetes.

2. The composition for use according to claims 1, wherein the gut microbiota uricase metabolic capacity is enhanced in the presence of uric acid.

3. The composition for use according to any one of claims 1-2 wherein the at least one bacterial strain with xanthine oxidase inhibitory activity expresses a metabolite that inhibits xanthine oxidase, wherein the metabolite is preferably a flavonoid.

4. The composition for use of any one of claims 1-3 wherein the plurality of bacterial strains has a synergistic effect in lowering serum urate levels.

5. The composition for use according to any preceding claim in form of a pharmaceutical composition or nutraceutical composition.

6. The composition for use according to claim 5 wherein the pharmaceutical composition or nutraceutical composition is co-formulated and/or co-administered with one or more micronutrients, preferably one or more vitamins selected from vitamin B2, vitamin B9 and vitamin C, and/or one or more amino acids, preferably arginine and/or citrulline, and/or one or more prebiotics preferably selected from fructooli-gosaccharides (FOS), inulins, galactooligosaccharides (GOS), resistant starch, pectin, beta-glucans, and xylooligosaccharides.

7. The composition for use according to claim 5 or 6 wherein the pharmaceutical composition or nutraceutical composition is administered in combination with a further agent, e.g. a therapeutic agent, and/or therapy, preferably with a second agent or therapy for the treatment of cardiovascular disease, metabolic syndrome, non-alcoholic fatty liver disease, chronic kidney disease, gout, insulin resistance, hypertension, hyperuricemia, dyslipidaemia, renal insufficiency, obesity, pre-diabetes and diabetes.

8. A composition for use according to any one of claims 5 to 7 wherein the pharmaceutical composition or nutraceutical composition is administered in combination with a xanthine oxidase inhibitor and/or a uricosuric agent, preferably probenecid, benzbromarone, sulfinpyrazone, allopurinol or febuxostat.

## Patentansprüche

1. Zusammensetzung, umfassend eine Vielzahl von Bakterienstämmen zur Verwendung in einem Verfahren zur Behandlung oder Vorbeugung des Zustands eines erhöhten Serumuratspiegels, wobei die Vielzahl von Bakterienstämmen aus den folgenden Stämmen ausgewählt ist: *Lactobacillus* casei-Stamm, hinterlegt bei der DSMZ unter der Hinterlegungsnummer DSM 33579 am 14. Juli 2020, *Lactobacillus plantarum*-Stamm, hinterlegt bei der DSMZ unter der Hinterlegungsnummer DSM 33580 am 14. Juli 2020, und *Lactobacillus plantarum-*Stamm, hinterlegt bei der DSMZ unter der Hinterlegungsnummer DSM 33581 am 14. Juli 2020, wobei die Vielzahl von Bakterienstämmen mindestens einen Bakterienstamm mit Xanthinoxidase-hemmender Aktivität und mindestens einen Bakterienstamm mit Uricase-Aktivität umfasst, wobei die Zusammensetzung die Uricase-Stoffwechselkapazität der Darmmikrobiota erhöht und die Xanthinoxidase-Stoffwechselkapazität verringert, wobei der Zustand eines erhöhten Serumuratspiegels aus Herz-Kreislauf-Erkrankungen, metabolischem Syndrom, nichtalkoholischer Fettlebererkrankung, chronischer Nierenerkrankung, Gicht, Insulinresistenz, Hypertonie, Hyperurikämie, Dyslipidämie, Niereninsuffizienz, Adipositas, Prädiabetes und Diabetes ausgewählt ist.

2. Die Zusammensetzung zur Verwendung gemäss den Ansprüchen 1, wobei die Uricase-Stoffwechselkapazität der Darmmikrobiota in Gegenwart von Harnsäure erhöht wird.

3. Die Zusammensetzung zur Verwendung gemäss einem der Ansprüche 1-2, wobei der mindestens eine Bakterienstamm mit Xanthinoxidase-hemmender Aktivität einen Metaboliten exprimiert, der Xanthinoxidase hemmt, wobei der Metabolit vorzugsweise ein Flavonoid ist.

4. Die Zusammensetzung zur Verwendung gemäss einem der Ansprüche 1-3, wobei die Vielzahl von Bakterienstämmen eine synergistische Wirkung bei der Senkung der Serumuratspiegel aufweist.

5. Die Zusammensetzung zur Verwendung gemäss einem der vorangehenden Ansprüche in Form einer pharmazeutischen Zusammensetzung oder einer nutrazeutischen Zusammensetzung.

6. Die Zusammensetzung zur Verwendung gemäss Anspruch 5, wobei die pharmazeutische Zusammensetzung oder die nutrazeutische Zusammensetzung mit einem oder mehreren Mikronährstoffen, vorzugsweise einem oder mehreren Vitaminen, ausgewählt aus Vitamin B2, Vitamin B9 und Vitamin C, und/oder einer oder mehreren Aminosäuren, vorzugsweise Arginin und/oder Citrullin, und/oder einem oder mehreren Präbiotika, vorzugsweise ausgewählt aus Fructooligosacchariden (FOS), Inulinen, Galactooligosacchariden (GOS), resistenter Stärke, Pektin, Beta-Glucanen und Xylooligosacchariden koformuliert und/oder gemeinsam verabreicht wird.

7. Die Zusammensetzung zur Verwendung gemäss Anspruch 5 oder 6, wobei die pharmazeutische Zusammensetzung oder die nutrazeutische Zusammensetzung in Kombination mit einem weiteren Mittel, z. B. einem therapeutischen Mittel, und/oder einer Therapie, verabreicht wird, vorzugsweise mit einem zweiten Mittel oder einer Therapie zur Behandlung von Herz-Kreislauf-Erkrankungen, metabolischem Syndrom, nichtalkoholischer Fettlebererkrankung, chronischer Nierenerkrankung, Gicht, Insulinresistenz, Hypertonie, Hyperurikämie, Dyslipidämie, Niereninsuffizienz, Adipositas, Prädiabetes und Diabetes.

8. Zusammensetzung zur Verwendung gemäss einem der Ansprüche 5 bis 7, wobei die pharmazeutische Zusammensetzung oder die nutrazeutische Zusammensetzung in Kombination mit einem Xanthinoxidasehemmer und/oder einem Urikosurikum, vorzugsweise Probenecid, Benzbromaron, Sulfinpyrazon, Allopurinol oder Febuxostat, verabreicht wird.

## Revendications

1. Composition comprenant une pluralité de souches bactériennes destinées à être utilisées dans un procédé de traitement ou de prévention d'un état de taux sériques d'urate élevés, dans laquelle la pluralité de souches bactériennes est choisie parmi les souches suivantes : la souche *Lactobacillus casei* déposée auprès de la DSMZ sous le numéro de dépôt DSM 33579 le 14 juillet 2020, la souche *de Lactobacillus plantarum* déposée auprès du DSMZ sous le numéro de dépôt DSM 33580 le 14 juillet 2020 et la souche *de Lactobacillus plantarum* déposée auprès du DSMZ sous le numéro de dépôt DSM 33581 le 14 juillet 2020, dans laquelle la pluralité de souches bactériennes comprend au moins une souche bactérienne présentant une activité inhibitrice de la xanthine oxydase et au moins une souche bactérienne présentant une activité uricase, dans laquelle la composition augmente la capacité métabolique de l'uricase du microbiote intestinal et réduit la capacité métabolique de la xanthine oxydase, dans laquelle l'état de taux sériques d'urate élevés est choisi parmi les maladies cardiovasculaires, le syndrome métabolique, la stéatose hépatique non alcoolique, les maladies rénales chroniques, la goutte, l'insulinorésistance, l'hypertension, l'hyperuricémie, la dyslipidémie, l'insuffisance rénale, l'obésité, le prédiabète et le diabète.

2. Composition pour utilisation selon les revendications 1, dans laquelle la capacité métabolique de l'uricase du microbiote intestinal est renforcée en présence d'acide urique.

3. Composition pour utilisation selon l'une quelconque des revendications 1 à 2, dans laquelle la au moins une souche bactérienne présentant une activité inhibitrice de la xanthine oxydase exprime un métabolite qui inhibe la xanthine oxydase, le métabolite étant de préférence un flavonoïde.

4. Composition pour utilisation selon l'une quelconque des revendications 1 à 3, dans laquelle la pluralité de souches bactériennes a un effet synergique dans la réduction des taux sériques d'urate.

5. Composition pour utilisation selon l'une quelconque des revendications précédentes, sous la forme d'une composition pharmaceutique ou d'une composition nutraceutique.

6. Composition pour utilisation selon la revendication 5, dans laquelle la composition pharmaceutique ou la composition nutraceutique est coformulée et/ou co-administrée avec un ou plusieurs micronutriments, de préférence une ou plusieurs vitamines choisies parmi la vitamine B2, la vitamine B9 et la vitamine C, et/ou un ou plusieurs acides aminés, de préférence l'arginine et/ou la citrulline, et/ou un ou plusieurs pré-biotiques choisis de préférence parmi les fructo-oligosaccharides (FOS), les inulines, les galacto-oligosaccharides (GOS), l'amidon résistant, la pectine, les bêta-glucanes et les xylo-oligosaccharides.

7. Composition pour utilisation selon la revendication 5 ou 6, dans laquelle la composition pharmaceutique ou nutraceutique est administrée en association avec un agent supplémentaire, par exemple un agent thérapeutique, et/ou un traitement, de préférence avec un deuxième agent ou traitement destiné au traitement des maladies cardiovasculaires, du syndrome métabolique, de la stéatose hépatique non alcoolique, de la maladie rénale chronique, de la goutte, de la résistance à l'insuline, l'hypertension, l'hyperuricémie, la dyslipidémie, l'insuffisance rénale, l'obésité, le prédiabète et le diabète.

8. Composition pour utilisation selon l'une quelconque des revendications 5 à 7, dans laquelle la composition pharmaceutique ou nutraceutique est administrée en association avec un inhibiteur de la xanthine oxydase et/ou un agent uricosurique, de préférence le probénécide, la benzbromarone, la sulfinpyrazone, l'allopurinol ou le fébuxostat.
